# EUROPÄISCHE PATENTANMELDUNG

(11) **EP 0 669 116 A1**
(43) Veröffentlichungstag der Anmeldung: **30.08.1995**
(21) Anmeldenummer: 95250020.5
(22) Anmeldetag: 26.01.1995
(51) Int. Cl.: A61F 2/36, A61F 2/30

(54) **Oberschenkelteil für eine Hüftgelenk-Endoprothese**

(30) Priorität: 26.01.1994 DE 9401529 U
(71) Anmelder: ARTOS Medizinische Produkte GmbH, D-12277 Berlin (DE)
(72) Erfinder: Kranz, Curt Dr.Ing,, D 10825 Berlin (DE)
(74) Vertreter: Christiansen, Henning, Dipl.-Ing.

(57) **Zusammenfassung**

Schaftprothese (1), insbesondere Hüftgelenk-Endoprothese, zur zementfreien Implantation, mit einem sich nach distal verjüngenden Schaft (2), welcher im proximalen Bereich an seiner nach anterior bzw. posterior gerichteten Außenoberfläche mit einer Profilierung versehen ist, welche sich in Eintreibrichtung erstreckende Ausnehmungen (4) in Form von Längsnuten aufweist, wobei mindestens ein Teil der Ausnehmungen (4) einen lichten Querschnitt aufweist, dessen jeweils innerhalb eines vorgegebenen Abstands zur Mittelachse bzw. Symmetrieebene gelegener Anteil nach proximal hin abnimmt.

## Beschreibung

Die Erfindung betrifft eine Hüftgelenk-Endoprothese der im Oberbegriff des Anspruchs 1 angegebenen Art.

Bei der Formgebung der Schaftteile von Hüftgelenk-Prothesen kommt es insbesondere bei der zementfreien Verankerung darauf an, eine innige Verbindung zwischen Schaftaußenfläche und angrenzendem Knochengewebe in denjenigen Bereichen zu erzielen, die hauptsächlich an der Krafteinleitung beteiligt sind. Hierbei muß einerseits auf die Elastizitätseigenschaften des Knochengewebes und andererseits auch auf dessen Festigkeit Rücksicht genommen werden. Die auftretenden Spannungen sollen weder erste vorgegebene Werte unterschreiten noch zweite vorgegebene Wert überschreiten. Fehleinstellungen führen zu Mikrobewegungen und Abbau des Knochengewebes, was seinerseits eine weitere Lockerung der Prothese zur Folge hat.

Aus der DE-PS 32 16 539 ist ein derartiger Oberschenkelteil einer Hüftgelenk-Endoprothese mit einem Kopfteil und einem zur zementfreien Verankerung vorgesehenen Schaft beschrieben. Der Schaft weist in seinem kopfnahen Bereich einen Schaftkern und eine zur Anlage an die Corticalis des Knochens bemessene Außenfläche und auf gegenüberliegenden Seiten mehrere, in Längsrichtung verlaufende Rippen auf, welche jeweils zwischen benachbarten Rippen Freiraum zur Aufnahme von Knochengewebe einschließen. Die konstante Breiten und Abstände aufweisenden Rippen nehmen in ihrer Höhe zum Konusende hin zu. Die starke Zunahme der Rippentiefe gestattet außerdem nicht die Konzeption einer für links- und rechtsseitige Verwendung gleichermaßen geeigneten symmetrischen Prothese, da aufgrund der physiologigischen Gegebenheiten und des zur Verfügung stehenden Knochenraums der Zuwachsbereich durch Ansätze in dorsaler Richtung begrenzt ist.

Die bekannte Prothese weist den Nachteil auf, daß beim Einbringen des Prothesenschafts in den vorbereiteten Oberschenkelknochen durch die vorhandene Rippenstuktur eine einseitig, im wesentlichen radial gerichtete Verdichtung der Spongiosa erfolgt, welche zu einer nicht vorhersehbaren Spannungsheraufsetzung - und damit insbesondere auch zu lokalen Belastungsspitzen - im Knochenmaterial führt. Die Form der Rippen erzeugt beim Eintreiben eine radiale Keilwirkung, welche bei unsachgemäßer Handhabung bis zu einer Längsspaltung des Femurs führen kann. Die genannte Wirkung wird auch bei normaler mechanischer Belastung durch ein Setzen der Prothese im Laufe der Zeit noch verstärkt und kann auch eine Lockerung des Prothesenschaftes mit allen damit verbundenen Komplikationen zur Folge haben.

Ausgehend von den Mängeln des Standes der Technik liegt der Erfindung die Aufgabe zugrunde, ein Oberschenkelteil der eingangs genannten Gattung zu entwickeln, welche ein "Setzen" des Schaftendes unter Heraufsetzung des Kraftschlusses zwischen Prothese und umgebendem Knochengewebe ohne Überbeanspruchung durch Keilwirkung ermöglicht.

Diese Aufgabe wird mit den kennzeichnenden Merkmalen des Anspruchs 1 gelöst.

Die Erfindung schließt die Erkenntnis ein, daß eine für die dauerhafte zementfreie Verankerung eines Prothesenschaftes die Spongiosa des den Schaft aufnehmenden Oberschenkelknochens in mehreren Richtungen - und nach Möglichkeit allmählich und gleichmäßig - verdichtet werden muß, um einer ungünstigen Spannungsverteilung beim Einbringen des Schaftes und bei der normalen mechanischen Belastung der implantierten Prothese vorzubeugen.

Da sich das Femur von proximal nach distal verjüngt, kann durch die Ausbildung einer an den Lateralflächen des Prothesenteils befindlichen Profilierung sowohl die zum Einwachsen des Knochenmaterials geschaffenen Oberflächenvergrößerung erreicht, als auch durch eine sich von proximal nach distal maßlich ändernde Profilierung die Art und Weise der proximalen Spongiosaverdichtung beim Einbringen des Prothesenschaftes in den Knochen gesteuert werden.

Mit den Maßnahmen nach der Erfindung ist es möglich, die Außenkontur der Prothese ohne wesentliches Übermaß an einen auszuräumenden Aufnahmebereich im Inneren des Femur anzupassen, wobei das "Setzen" der Prothese ohne wesentliches radiales Aufkeilen erfolgt, da sich diese lediglich in die vorbereitete Form verbessert einpaßt. Ein "Verkeilen" erfolgt bevorzugt in tangentialer Richtung, wobei diese Keilwirkung nicht zu einer die Knochenröhre aufweitenden Beanspruchung führt, sondern zu einer Knochenverdichtung, die jeweils zwischen benachbarten Rippen erfolgt, so daß die auftretenden Spannungen nicht von dem umgebenden Knochen, sondern unmittelbar von den benachbarten Rippen aufgenommen werden. Die Verdichtung bezieht sich daher auf Bereiche, welche bevorzugt nahe der einhüllenden Oberflächenkontur der Prothese gelegen sind. Die "Verdichtung" erfolgt also durch zunehmendes Verklemmen unter Einwirkung in tangentialer Richtung auf Bereiche, die beim Eintreiben oder Setzen des Schaftes von identischen Oberflächenbereichen der Rippenstruktur nacheinander passiert werden. Als Bezug für die Verdichtung des Querschnitts der Rippen im Vergleich zu der zwischen den Rippen verbleibenden lichten Flächenbereich können dabei auch die radialen Abstände der relevanten Bereiche von der Mittelachse der beim Eintreiben gemeinsam bewegten Teile der Prothese dienen, welche dann eine Bezugsebene für die "tangentiale Keilwirkung" - im Gegensatz zu der bekannten "radialen Keilwirkung" bildet.

Besonders vorteilhaft ist der Umstand, daß sich mit den erfinderischen Maßnahmen eine symmetrische Prothese für beidseitige Anwendung schaffen läßt, welche in ihrem Einpaßverhalten solchen Prothesenschäften angenähert ist, die bisher eine spezielle unsymmetrische Gestaltung mit den resultierenden gesteigerten Herstellungs- und Bevorratungskosten erforderten.

Entsprechend einer bevorzugten Ausführungsform der Erfindung weist der Oberschenkelteil einer Hüftgelenk-Endoprothese an den proximalen Breitseiten des Schaftes eine rippenförmige Profilierung auf, wobei die einzelnen Rippen im wesentlichen in Einführungsrichtung geradlinig verlaufen. Um die gewünschte Verdichtung der im Knocheninneren nach entsprechender Implantionsvorbereitung verbliebenen, insbesondere der proximalen, Spongiosa sowohl in radialer und in dazu im wesentlichen senkrechter Richtung weitgehend gleichmäßig zu erreichen, wird zumindest das Breitenmaß des Querschnittsprofils der einzelnen Rippen kontinuierlich von proximal nach distal vermindert. Dadurch wird in günstiger Weise errreicht, daß sich die wirksame Quersschnittsfläche der zwischen den Rippen befindlichen Ausnehmungen von distal nach proximal allmählich verringert.

Es ist nach einer vorteilhaften Weiterbildung der Erfindung fertigungstechnisch besonders günstig, wenn sich die das Querschnittsprofil der Rippen bestimmenden Abmessungen des durch Schmieden herstellbaren Oberschenkelteils im wesentlichen konusförmig verändern. Die Änderung erfolgt derart, daß sich die Querschnittsfläche der Ausnehmungen zwischen den einzelnen Rippen des Profils nach distal vergrößert, wogegen die Querschnittsfläche der Rippen nach distal abnimmt.

Als vorteilhafte Profilierung im Sinne der Aufgabe der Erfindung sind Rippen mit einer im wesentlichen dreieckförmigen, rechteckigen oder aus Bogenelementen gebildeten Querschnittsfläche an den proximalen Breitseiten des Prothesenschaftes vorgesehen. Die für die gewünschte SpongiosaVerdichtung erforderliche Verringerung der Querschnittsprofilfläche der Rippen (bzw. der Vergrößerung der Querschnittsfläche der zwischen den Rippen befindlichen Ausnehmungen) nach distal ist durch entsprechende Variation eines Höhen-und/oder Breitenmaßes oder eines Radius der die jeweilige Querschnittsfläche begrenzenden Linienzuges bzw. eines Winkelmaßes zwischen zwei der die Querschnittsfläche begrenzenden Linienzüge.

Entsprechend einer weiteren vorteilhaften Ausführungsform der Erfindung ist in dem Oberschenkelteil der Hüftgelenk-Endoprothese eine axiale, sich von proximal nach distal erstreckende Durchgangsbohrung vorgesehen. Dabei weist der Schaft der Prothese im proximalen Bereich an seiner proximalen Schmalseite und im distalen Bereich an seiner distalen Schmalseite jeweils eine V-förmige Kerbe auf. Die Kerben erstrecken sich jeweils bis zu der Längsbohrung des Schaftes.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 einen Längsschnitt durch eine bevorzugte Ausführungsform der Erfindung in schematisierter Darstellung,
Figur 2 die schematisierte Darstellung der Ansicht eines Schnittes längs der Linie C...C gemäß Figur 1,
Figur 3 die Draufsicht auf die in Figur 1 dargestellte Ausführungsform der Erfindung,
Figur 4 die Darstellung der Ansicht eines Schnittes längs der Linie B...B gemäß Figur 2,
Figur 5 die Darstellung einer Teilansicht des Schnittes längs der Linie D...D gemäß Figur 1,
Figur 5a die Darstellung einer Teilansicht des Schnittes längs der Linie E...E gemäß Figur 1 sowie
Figuren 6, 6a, 6b, 7, 7a, 8, 8a, 8b, 9, 9a, 9b, die Darstellung einer Teilansicht des Schnittes längs der Linie D...D bzw. einer Teilansicht des Schnittes längs der Linie E...E gemäß Figur 1 sowie eine perspektivische Darstellung eines profilierten Schaftabschnittes für verschiedene vorteilhafte Weiterbildungen der in Figur 1 gezeigten bevorzugten Ausführungsform der Erfindung.

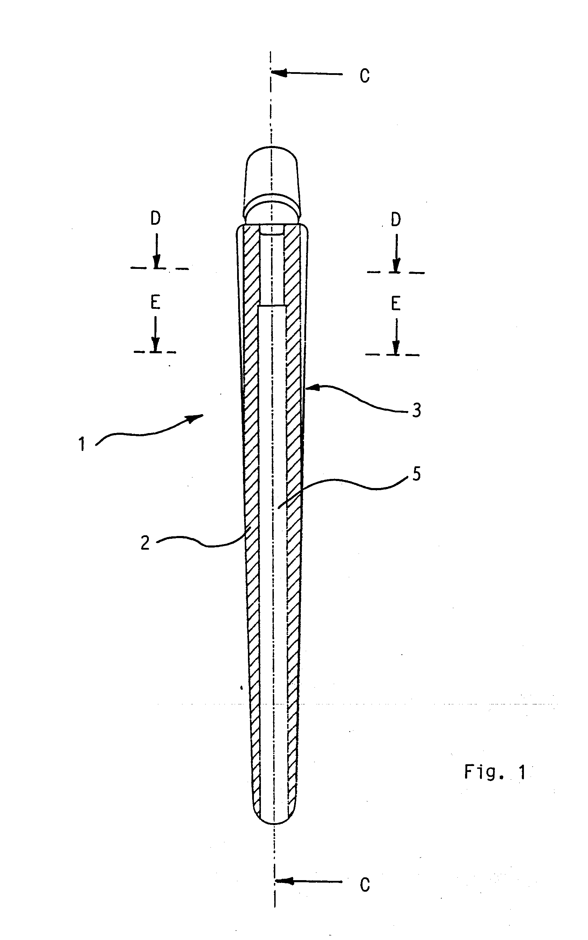

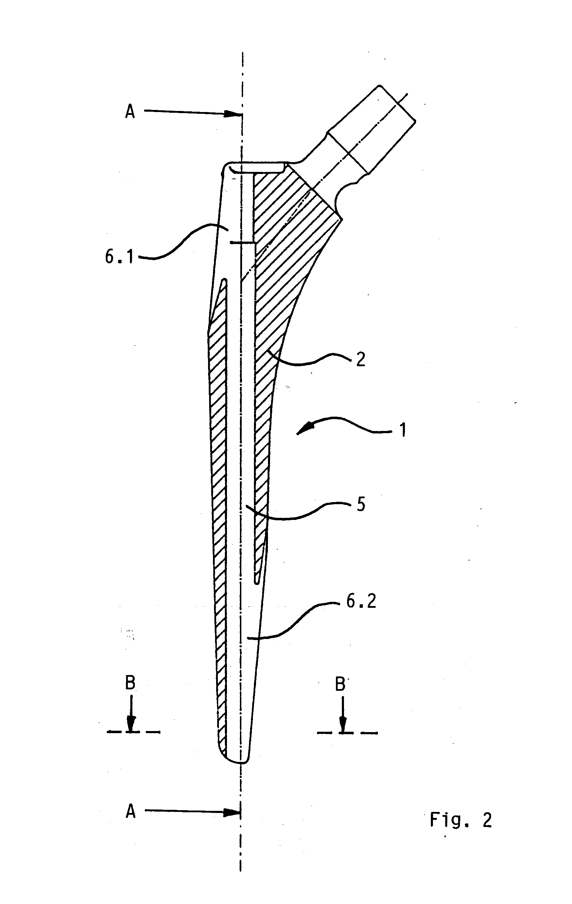

Der in den Figuren 1, 2 und 3 dargestellte schaftteil 1 einer Hüftgelenk-Endoprothese als Lateralschnitt (Figur 1 als Ansicht des Schnittes längs der Linie A...A gemäß Figur 2,Figur 2 als Ansicht des Längsschnittes längs der Linie C...C gemäß Figur 1) bzw. als Ansicht von oben. Die anatomisch optimierte Geometrie des Schaftes 2 des Oberschen-kelteils 1, die durch ovale Schaftquerschnitte im proximalen Bereich gekennzeichnet ist, gewährleistet bei den unterschiedlichsten Belastungsfällen der Hüftgelenk-Endoprothese eine optimale Krafteinleitung in den Oberschenkelknochen. Durch ein sich nach distal konisch verjüngendes Schaftende weist der Oberschenkelteil 1 eine genügende Elastizität, welche insbesondere durch die V-förmigen Kerben 6.1 und 6.2 erreicht wird. Die Kerbe 6.1 befindet sich in vorteilhafter Weise proximal ander proximalen Schmalseite und distal an der distalen Schmalseite des Schaftes 2. Die erforderliche Steifigkeit des Prothesenschaftes 2 wird durch eine Bohrung 5 erreicht, welche sich on proximal nach distal durch den Schaft erstreckt.

Sowohl an der vorderen als auch an der hinteren Seiten des sich proximal blattähnlich erweiternden Schaft 2 ist eine Profilierung vorgesehen, welche sich aus Rippen 3 und zwischen ihnen angeordneten Ausnehmungen 4 zusammensetzt.

Diese Rippenstruktur ist derart konzipiert, daß sich in proximaler Richtung der Abstand zwischen den einzelnen Rippen 3 verengt und die Rippenhöhe nach distal gleichzeitig reduziert wird. Die Verengung und die Höhenreduzierung wird in günstiger Weise konisch vorgenommen, da sich dadurch herstellungstechnische Vorteile ergeben. Durch die vorstehend beschriebene Rippenstruktur ist gewährleistet, daß bei einem nach dem Implantieren der Prothese eintretenden Setzen in vorteilhafter Weise ein sogenannter proximaler Pressfit mit metaphysärer Verankerung des Prothesenschaftes 2 erreicht wird. Das Formraspeln zur implantationsgerechtes Vorbereitung des Femurmarkraumes wird im Bereich der späteren Position der Rippenstruktur des Schaftes mit Untermaß durchgeführt. Dadurch findet hier in günstiger Weise eine primäre Verklemmung mit gleichzeitiger Verdichtung der Spongiosastruktur statt. Aufgrund der sich konisch sowohl in der Breite und als auch in der Höhe nach distal verjüngenden Rippen 3 erfolgt die Spongiosaverdichtung nicht nur in radialer Richtung , sondern gleichzeitig auch in einer dazu im wesentlichen senkrechten Richtung. Diese Gleichmäßigkeit der Verdichtung schafft in vorteilhafter Weise eine Festigkeitserhöhung ohne daß es zu einer zusätzlichen mechanischen Belastung der Corticalis kommt.

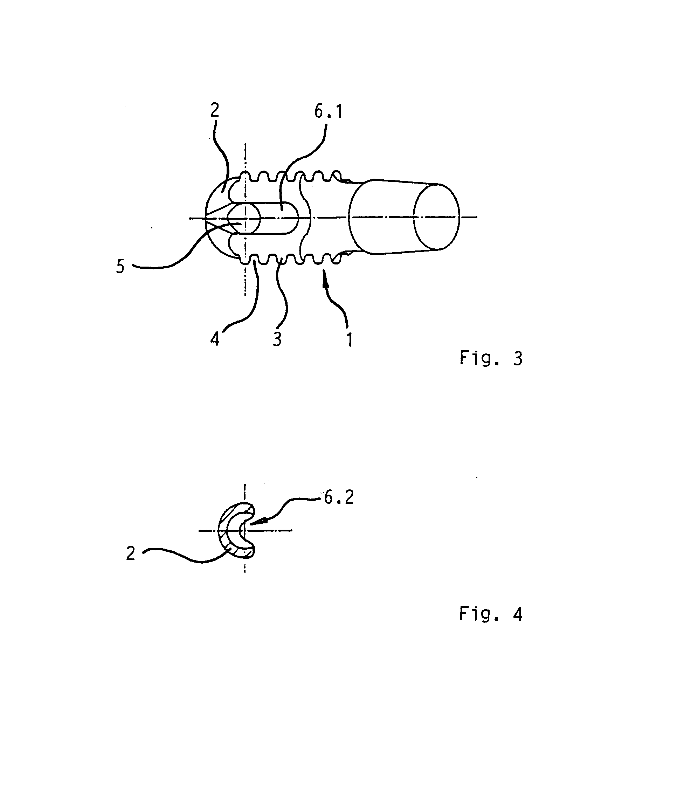

In Figur 4 ist die Ansicht eines Schnittes längs der Linie B...B gemäß Figur 2 dargestellt. Durch die V-förmige Kerbe 6.2, welche sich bis zum distalen Ende des Schaftes 2 erstreckt, besitzt der Schaft 2 eine spezielles Flächenträgheitsmoment, welches bei ausreichender Elastizität eine "sanfte" Kraftüberleitung in das Femur gewährleistet.

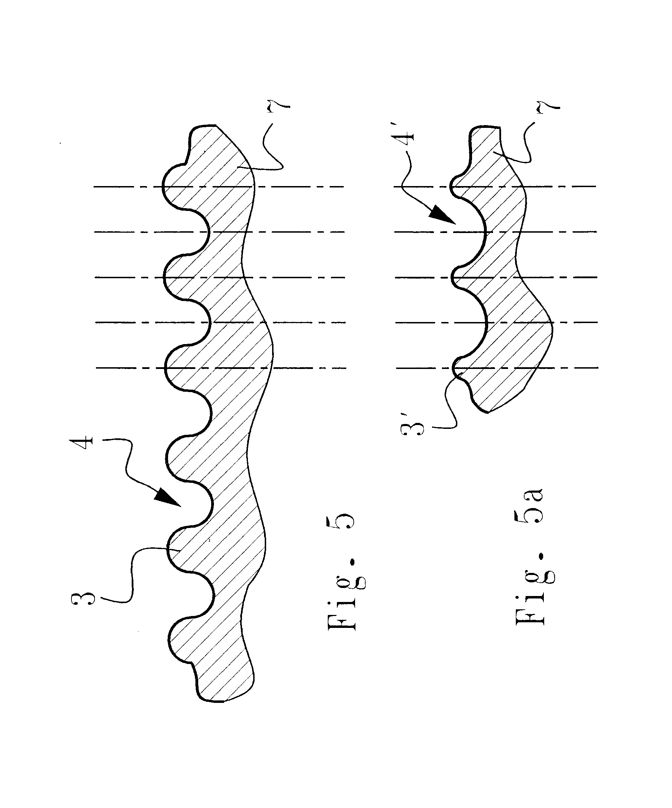

Die Figuren 5 und 5a zeigen die bevorzugte Ausführungsform der Profilierung im proximalen Bereich des Schaftes als Ansicht eines Teilschnittes längs der Linie D...D bzw. E...E gemäß Figur 1. Sowohl die Rippen 3 der Profilierung des Wandabschnittes 7 des Schaftes als auch die zwischen den Rippen 3 vorhandenen Ausnehmungen 4 sind von Kreisbogenteilen begrenzt (Figur 5). Um die gewünschte Verdichtung der Spongiosa im Oberschenkelknochen zu erreichen, wird die Querschnittsfläche der Ausnehmungen 4' in distaler Richtung vergrößert (Figur 5a). Die Querschnittsfläche der Rippen 3' verringert sich entsprechend. Die grundsätzliche Form der Ausnehmungen und der Rippen bleibt erhalten. Der Radius der Rippen verringert sich im Verhältnis der Radiusvergrößerung der Ausnehmungen je Längeneinheit langsamer.

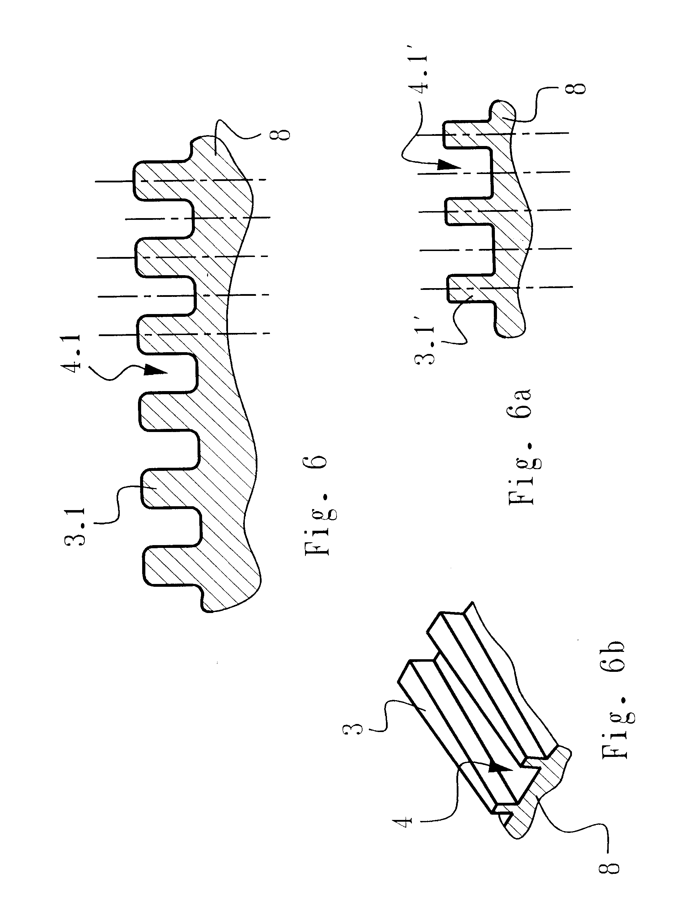

In den Figuren 6, 6a, 6b, 7, 7a, 8, 8a, 8b, 9, 9a und 9b sind verschiedene, vorteilhafte Ausbildungsformen der Profilierung der proximalen Breitseiten des Oberschenkelteils einer Hüftgelenk-Endoprothese als Ansicht eines Teilschnittes durch die Wandung 8, 9, 10, 11 im proximalen Bereich des Schaftes 2 längs der Linien D...D bzw. E...E. Allen Profilierungen ist gemein, daß die Querschnittsfläche der Ausnehmungen 4.1, 4.2, 4.3, 4.4 bzw. 4.1', 4.2', 4.3', 4.4' vom proximal nach distal kontinuierlich, vorzugsweise konisch, zunimmt. Gleichzeitig wird die Höhe der Profilierungsrippen 3.1, 3.2, 3.3, 3.4 bzw. 3.1', 3.2', 3.3', 3.4' kontinuierlich reduziert. Die durch die daraus resultierende Keilwirkung führt in vorteilhafter Weise nicht nur zu einer Verdichtung der Spongiosa in radialer Richtung, sondern auch in einer dazu senkrech-ten Richtung und gewährleistet einen festen Sitz der Hüftgelenk-Endoprothese im Oberschenkelknochen

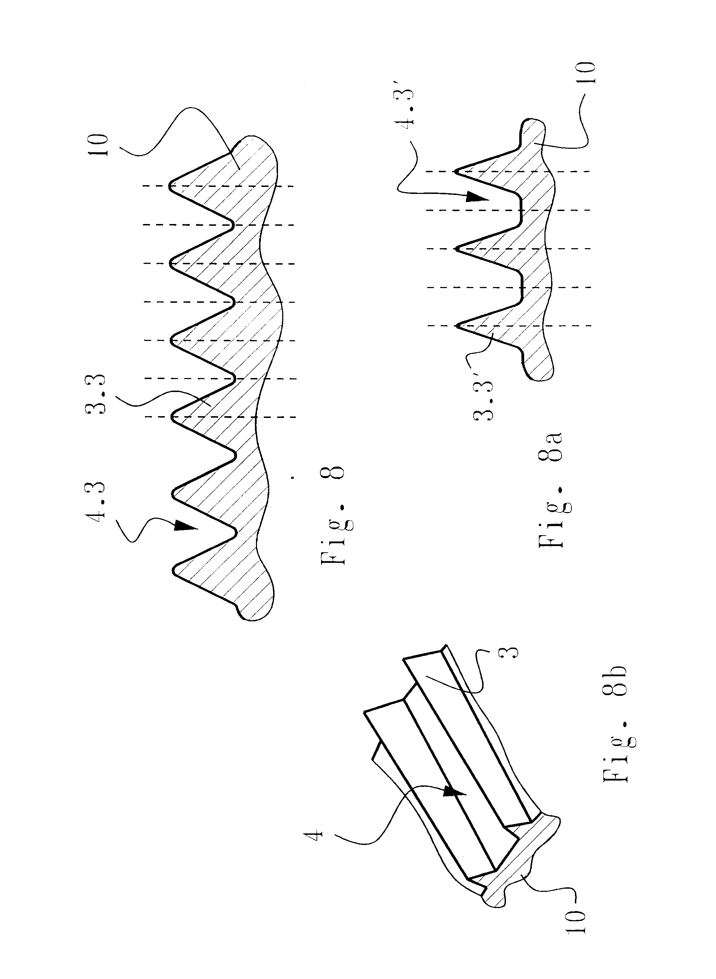

Führ die Querschnittsfläche der Profilierungsrippen bzw. der Ausnehmungen ist eine rechteckige (Positionen 3.1, 4.1 bzw. 3.1', 4.1' in den Figuren 6, 6a) oder eine im wesentlichen dreieckförmige Ausbildung (Positionen 3.3, 4.3 bzw. 3.3', 4.3' in den Figuren 8, 8a) günstig. Die gewünschte Änderung der Querschnittsflächen läßt sich auf einfache Weise durch Variation des Höhen/Seiten-Verhältnis eines Rechtecks oder durch Veränderung des Scheitelwinkels bzw. der Höhe eines Dreiecks erreichen. In den Figuren 6b und 8b sind entsprechende Profilabschnitte perspektivisch dargestellt.

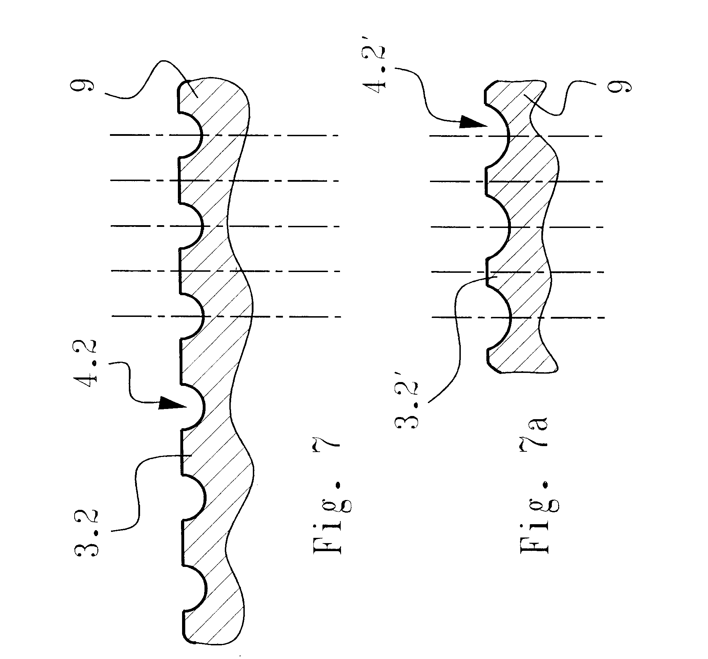

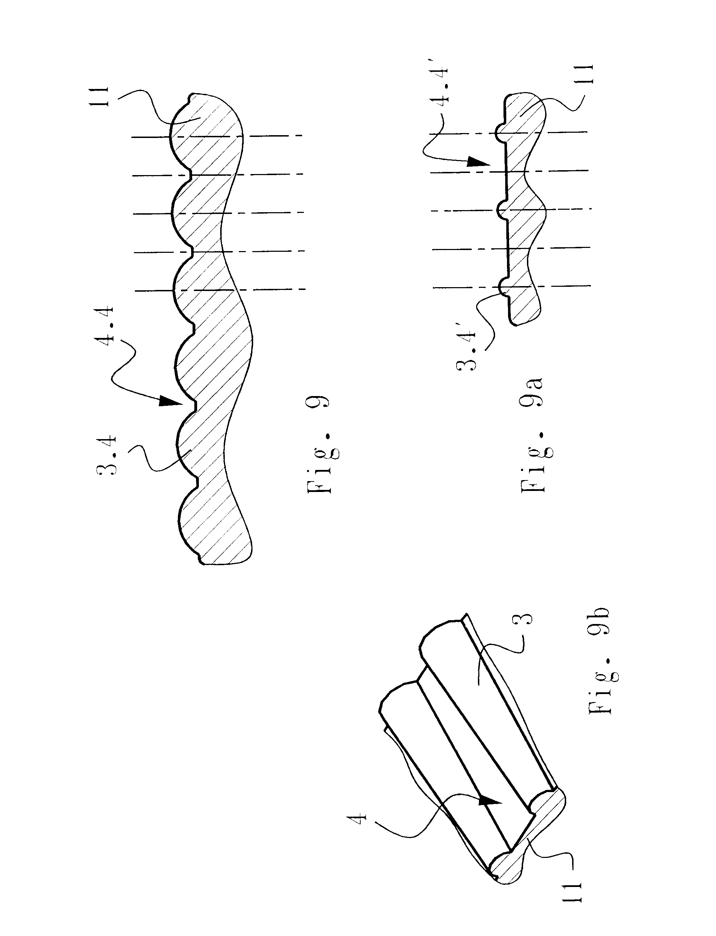

Bei im wesentlichen durch Bogenabschnitte begrenzten Querschnittsflächen der Ausnehmungen (Positionen 4.2, 4.2' in den Figuren 7, 7a) bzw. der Profilierungsrippen (Positionen 3.4, 3.4' in den Figuren 9, 9a) wird eine Veränderung der entsprechenden Radien von proximal nach distal vorgenommen. Figur 9a zeigt in perspektivischer Darstellung einen Schaftabschnitt mit bogenförmig begrenzten Rippen.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

## Patentansprüche

1. Schaftprothese, insbesondere Hüftgelenk-Endoprothese, zur zementfreien Implantation, mit einem sich nach distal verjüngenden Schaft, welcher im proximalen Bereich an seiner nach anterior bzw. posterior gerichteten Außenoberfläche mit einer rippenförmigen Profilierung versehen ist, welche sich in Eintreibrichtung erstreckende Ausnehmungen in Form von Längsnuten aufweist,
dadurch gekennzeichnet,
daß das Verhältnis des Materialquerschnitts der Rippen zum zwischen den Rippen verbleibendem lichtem Querschnitt in proximaler Richtung zunimmt, so daß die Klemmwirkung der Rippen im umgebenden Knochenraum beim Eintreiben der Prothese in distaler Richtung zu-, dagegen beim Ausziehen der Prothese in distaler Richtung abnimmt.

2. Schaftprothese nach Anspruch 1, dadurch gekennzeichnet, daß dieses Verhältnis sich bezieht auf Bereiche von Rippen und lichtem Querschnitt, die von der äußeren umhüllenden Kontur der Prothese gleiche Abstände bzw. vom Zentrum gleiche radiale Abstände aufweisen.

3. Schaftprothese nach einem der vorangehenden Ansprüche, dadurch gekennzeichnet, daß die Verringerung des Querschnitts der die lichten Bereiche bildenen Ausnehmungen nach Proximale durch Verringerung der Breite der Längsnuten, durch Verringerung des Spitzenwinkels bei drei- oder mehreckiger Ausgestaltung oder Verringerung des Radius von konkaven Verrundungen bzw. durch Vergrößerung des Radius von konvexen Verrundungen erfolgt.

4. Schaftprothese nach einem der vorangehenden Ansprüche, dadurch gekennzeichnet, daß der Querschnitt der Ausnehmungen im wesentlichen kontinuierlich abnimmt.

5. Schaftprothese nach einem der vorangehenden Ansprüche, dadurch gekennzeichnet, daß die Abnahme des lichten Querschnitts im wesentlichen unter Beibehalten der gewählten Profilform der Längsrippen (3, 3.1, 3.2, 3.3, 3.4) erfolgt.

6. Schaftprothese nach einem der vorangehenden Ansprüche, dadurch gekennzeichnet, daß die Profilierung an der nach anterior und an der nach posterior gerichteten Seite des proximalen Schaftbereichs gleichartig, insbesondere spiegelsymmetrisch, ausgebildet sind.

7. Schaftprothese nach einem der Ansprüche 1 bis 5, dadurch gekennzeichnet, daß das Querschnittsprofil der Längsrippen (3.1) im wesentlichen rechteckförmig ausgebildet ist.

8. Schaftprothese nach Anspruch 7, dadurch gekennzeichnet, daß eine Flächenreduzierung des Querschnittsprofils nach distal durch Verringerung der Rechteckbreite oder der Rechteckbreite und -höhe erfolgt.

9. Schaftprothese nach einem der vorangehenden Ansprüche, dadurch gekennzeichnet, daß das Querschnittsprofil der Längsrippen (3) durch konkave und konvexe Bogenabschnitte bestimmt wird, welche in Reihenfolge abwechselnd angeordnet sind.

10. Schaftprothese nach einem der vorangehenden Ansprüche, dadurch gekennzeichnet, daß der Schaft eine Längsbohrung aufweist.

11. Schaftprothese nach einem der vorangehenden Ansprüche, dadurch gekennzeichnet, daß der Schaft im proximalen Bereich an seiner lateralen Schmalseite und im distalen Bereich an seiner medialen Schmalseite jeweils eine, insbesondere eine V-förmige Ausnehmung, aufweist.

12. Schaftprothese nach Anspruch 11, dadurch gekennzeichnet, daß sich die Ausnehmungen sich bis zur Längsbohrung des Schaftes erstrecken.
